# EUROPÄISCHE PATENTSCHRIFT

(11) **EP 0 776 658 B1**
(45) Veröffentlichungstag und Bekanntmachung des Hinweises auf die Patenterteilung: **16.02.2000**
(21) Anmeldenummer: 96118237.5
(22) Anmeldetag: 14.11.1996
(51) Int. Cl.: A61K 7/50, A61K 7/48

(54) **Zuckertenside und Fettsäurepartialglyceride enthaltende kosmetische und/oder pharmazeutische Zubereitungen**
Sugartensides and fatty acid partial glycerides containing cosmetical and/or pharmaceutical preparations
Préparations cosmétiques et/ou pharmaceutiques à base de tensioactifs saccharides et de glycérides partiels d'acides gras

(30) Priorität: 23.11.1995 DE 19543633
(43) Veröffentlichungstag der Anmeldung: 04.06.1997
(73) Patentinhaber: Cognis Deutschland GmbH, 40589 Düsseldorf (DE)
(72) Erfinder: Hensen, Hermann, Dr., 42781 Haan (DE); Boyxen, Norbert, 47906 Kempen (DE); Rottmann, Mirella, 40231 Düsseldorf (DE); Seipel, Werner, 40723 Hilden (DE); Mohr, Klaus-Michael, 42327 Wuppertal (DE)

(56) Entgegenhaltungen:
- EP-A- 0 507 047
- WO-A-92/07543
- WO-A-96/14374
- DE-A- 4 139 935

## Beschreibung

Die Erfindung betrifft Zubereitungen mit einem Gehalt an ausgewählten Zuckertensiden und ausgewählten Fettsäurepartialglyceriden sowie deren Verwendung als Rückfettungsmittel zur Herstellung von kosmetischen bzw. pharmazeutischen Produkten.

### Stand der Technik

Zubereitungen, die zur Reinigung und Pflege der menschlichen Haut und der Haare eingesetzt werden, enthalten in der Regel ein oder mehrere oberflächenaktive Substanzen, insbesondere auf Basis von anionischen oder amphoteren Tensiden. Da die alleinige Verwendung von Tensiden Haut und Haare zu sehr austrocknen würde, ist es im allgemeinen üblich, solchen Mitteln rückfettende Substanzen zuzusetzen. Es liegt dabei auf der Hand, daß diese Stoffe nicht nur eine hinreichende rückfettende Wirkung, sondern den Erfordernissen des Marktes folgend gleichzeitig auch eine optimale dermatologische Verträglichkeit aufweisen müssen.

Aus der Deutschen Patentschrift **DE-C2 41 39 935** (Kao) sind flüssige Körperreinigungsmittel auf wäßriger Basis bekannt, die 5 bis 35 Gew.-% anionische Tenside, 2,5 bis 15 Gew.-% Alkylpolyglucoside und 0,5 bis 15 Gew.-% gesättigte Fettsäuremonoglyceride mit 8 bis 18 Kohlenstoffatomen im Fettacylrest aufweisen. Die in diesem Dokument vorgeschlagenen Monoglyceride weisen jedoch auch in Abmischung mit Glucosiden eine nicht ausreichende dermatologische Verträglichkeit auf. Zudem sind die Mischungen in Abwesenheit von Wasser in der Regel fest und lassen sich daher nicht kalt verarbeiten. Gemäß der Lehre der Deutschen Patentanmeldung **DE-A 27 01 266** sind Schaumbadzusammensetzungen bekannt, die 1 bis 50 Gew.-% Fettsäuremonoglyceride und 5 bis 50 Gew.-% Alkylsulfate, Alkylethersulfate und/oder Ethercarbonsäuresalze enthalten können. Diese Mischungen weisen jedoch keine vorteilhaften rückfettenden Eigenschaften auf. Gegenstand der Europäischen Patentschrift **EP-B1 0 554 292** (Henkel) sind O/W-Emulsionen, die Ölkörper, Alkylpolyglucoside, Fettsäurepartialglyceride und gegebenenfalls Fettalkohole enthalten. Auch diese Mischungen sind hinsichtlich ihrer rückfettenden Wirkung, ihrer Hautverträglichkeit und Konsistenz in wasserfreiem Zustand nicht vollkommen zufriedenstellend. Aus der Europäischen Patentanmeldung **EP-A1 0 538 762** (Kao) sind schließlich Haarbehandlungsmittel bekannt, die kationische Tenside, Alkylpolyglucoside und Ölkörper, beispielsweise auch Fettsäuremonoglyceride enthalten.

Die Aufgabe der Erfindung hat somit darin bestanden, ethylenoxidfreie Rückfettungsmittel zur Verfügung zu stellen, die bei Raumtemperatur flüssig sind, verdickende Eigenschaften aufweisen und insbesondere eine besonders hohe dermatologische Verträglichkeit aufweisen.

### Beschreibung der Erfindung

Gegenstand der Erfindung sind kosmetische und/oder pharmazeutische Zubereitungen, enthaltend
(a) 0,5 bis 70, vorzugsweise 20 bis 60 Gew.-% Zuckertenside ausgewählt aus der Gruppe, die gebildet wird von (a1) Alkyl- und/oder Alkenyloligoglykosiden und/oder (a2) Fett-säure-N-alkylpolyhydroxyalkylamiden, und
(b) 0,5 bis 40, vorzugsweise 25 bis 35 Gew.-% Fettsäurepartialglyceride ausgewählt aus der Gruppe, die gebildet wird von Ölsäuremonoglycerid, Ölsäurediglycerid, Isostearinsäuremonoglycerid, Isostearinsäurediglycerid, Behensäuremonoglycerid, Behensäurediglycerid, Isobehensäuremonoglycerid und/oder Isobehensäurediglycerid,
mit der Maßgabe, daß sich die Mengenangaben gegebenenfalls mit weiteren Hilfs- und Zusatzstoffen und/oder Wasser zu 100 Gew.-% ergänzen.

Überraschenderweise wurde gefunden, daß Mischungen der genannten Zuckertenside mit den ausgewählten Partialglyceriden eine besonders hohe dermatologische Verträglichkeit bei optimalen rückfettenden Eigenschaften aufweisen. Die Mischungen sind zudem bei Raumtemperatur flüssig, so daß sie sich auch kalt verarbeiten lassen. Ein weiterer Vorteil besteht darin, daß die Mischungen in tensidischen Systemen eine Viskosität aufbauen.

### Alkyl- und/oder Alkenyloligoglykoside

Alkyl- und Alkenyloligoglykoside, die die Zuckertensidkomponente (a1) ausmachen, stellen bekannte nichtionische Tenside dar, die der Formel **(I)** folgen,

**R**^{**1**}**O-[G]**_{**p**} **(I)**

in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden, beispielsweise durch säurekatalysierte Acetalisierung von Glucose mit Fettalkoholen.

Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligo**glucoside**. Die Indexzahl p in der allgemeinen Formel **(I)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomeri-sierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.

Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

### Fettsäure-N-alkylpolyhydroxyalkylamide

Fettsäure-N-alkylpolyhydroxyalkylamide, die Zuckertensidkomponente (a2) ausmachen, stellen nichtionische Tenside dar, die der Formel **(II)** folgen, 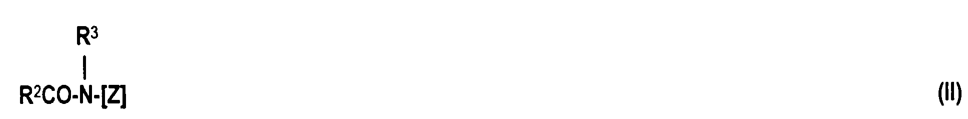 in der R²CO für einen aliphatischen Acylrest mit 6 bis 22 Kohlenstoffatomen, R³ für einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

Bei den Fettsäure-N-alkylpolyhydroxyalkylamiden handelt es sich um bekannte Stoffe, die üblicherweise durch reduktive Aminierung eines reduzierenden Zuckers mit einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften **US 1,985,424, US 2,016,962** und **US 2,703,798** sowie die Internationale Patentanmeldung **WO 92/06984** verwiesen. Eine Übersicht zu diesem Thema von H.Kelkenberg findet sich in **Tens. Surf. Det. 25, 8 (1988)**. Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher **Fettsäure-N-alkylglucamide** dar, wie sie durch die Formel (III) wiedergegeben werden:

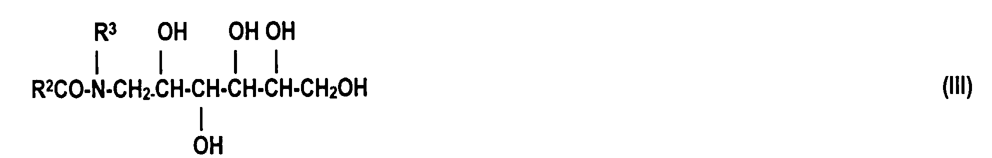

Vorzugsweise werden als Fettsäure-N-alkylpolyhydroxyalkylamide Glucamide der Formel **(III)** eingesetzt, in der R³ für eine Alkylgruppe steht und R²CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel **(III)**, die durch reduktive Aminierung von Glucose mit Methylamin und anschließende Acylierung mit Laurinsäure oder C_{12/14}-Kokosfettsäure bzw. einem entsprechenden Derivat erhalten werden. Weiterhin können sich die Polyhydroxyalkylamide auch von Maltose und Palatinose ableiten.

### Fettsäurepartialglyceride

Bei den ausgewählten Fettsäurepartialglyceriden, die die Komponente (b) ausmachen, handelt es sich um bekannte Stoffe, die nach den einschlägigen Verfahren der präparativen organischen Chemie hergestellt werden können. Fettsäurepartialglyceride, d.h. in der Regel technische Mischungen von Mono- und Diglyceriden werden üblicherweise durch Umesterung der entsprechenden Triglyceride mit Glycerin oder durch gezielte Veresterung von Fettsäuren erhalten. Die Abtrennung von nichtumgesetzten Ausgangsstoffe sowie die Anreicherung von Monoglyceriden in den Gemischen erfolgt in der Regel über eine Molekulardestillation. Die Partialglyceride der vorliegenden Erfindung werden vorzugsweise durch Veresterung von Glycerin mit Ölsäure, Isostearinsäure, Behensäure oder Isobehensäure hergestellt. Bei der Isobehensäure handelt es sich um die gehärtete Monomerfraktion, die bei der Dimerisierung von Erucasäure anfällt. Die Erfindung schließt die Erkenntnis ein, daß technische Mono/Diglycerid-Mischungen in der Anwendung eine noch bessere dermatologische Verträglichkeit als die reinen Monoglyceride zeigen. Demzufolge sind solche technischen Fettsäuremono-/diglyceride bevorzugt, die eine molares Verhältnis von Mono- zu Diester im Bereich von 10 : 90 bis 90 : 10 und insbesondere 80 : 20 bis 50 : 50 aufweisen.

### Tenside

Die erfindungsgemäßen Zubereitungen können 0,5 bis 40 und vorzugsweise 1 bis 25 Gew.-% anionische und/oder amphotere bzw. zwitterionische Tenside enthalten. Typische Beispiele für **anionische Tenside** sind Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, Acyllactylate, Acyltartrate, Acylglutamate, Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124** oder **J.Falbe (ed.), "Katalysatoren, Tenside und Mineralöladditive", Thieme Verlag, Stuttgart, 1978, S. 123-217** verwiesen.

### Ölkörper

Die erfindungsgemäßen Zubereitungen können 0,5 bis 25 und vorzugsweise 1 bis 15 Gew.-% Ölkörper enthalten. Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimerdiol) und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate, Dialkylether, Siliconöle und/oder aliphatische bzw. naphthenische Kohlenwasserstoffe in Betracht.

### Zubereitungen

Eine typische rückfettende Zubereitung, die den Gegenstand der Erfindung illustrieren soll, kann beispielsweise eine Zusammensetzung gemäß Tabelle 1 aufweisen:

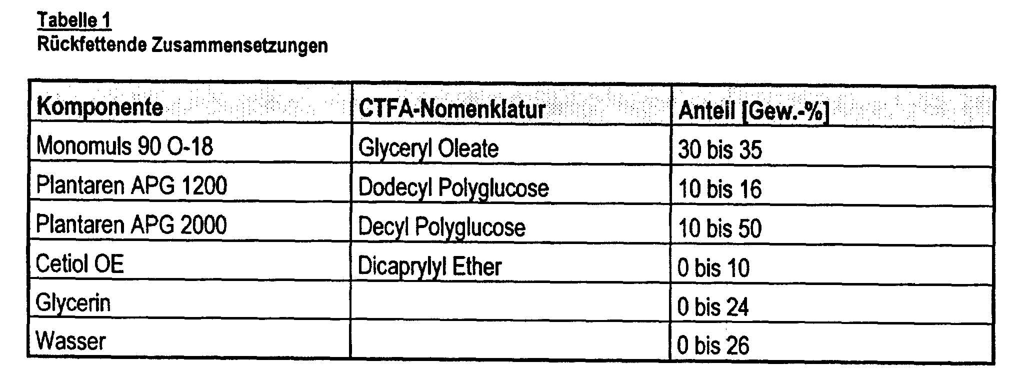

Falls erforderlich, können die erfindungsgemäßen Zubereitungen jeweils 0,5 bis 25 und vorzugsweise 1 bis 15 Gew.-% Glycerin und/oder Wasser enthalten.

### Gewerbliche Anwendbarkeit

Die erfindungsgemäßen Zubereitungen zeichnen sich durch eine hohe dermatologische Verträglichkeit und ausgezeichnete rückfettende Eigenschaften aus. Sie sind flüssig und pumpbar und lassen sich daher kalt verarbeiten. Sie sind frei von Stickstoff und Ethylenoxid und auch in Abwesenheit von Konservierungsmitteln gegen mikrobiellen Befall ausreichend stabilisiert. Sie weisen verdickende Eigenschaften auf und sind dabei vollständig biologisch abbaubar.

Ein weiterer Gegenstand der Erfindung betrifft daher ihre Verwendung als Rückfettungsmittel zur Herstellung von kosmetischen und/oder pharmazeutischen Produkten, beispielsweise für den Bereich der Haar- und Körperpflege.

Die genannten Pflegemittel, wie beispielsweise Haarshampoos, Duschbäder, Schaumbäder, Tagescremes, Nachtcremes, Pflegecremes, Nährcreme, Bodylotions, Salben und dergleichen, können als weitere Hilfs- und Zusatzstoffe Emulgatoren, kationische Polymere, Siliconverbindungen, Überfettungsmittel, Fette, Wachse, Stabilisatoren, biogene Wirkstoffe, Konservierungsmittel, Farb- und Duftstoffe enthalten.
Als **Emulgatoren** kommen beispielsweise nichtionogene Tenside aus mindestens einer der folgenden Gruppen in Frage:
(b1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(b2) C_{12/18}-Fettsäuremono- und -diester von Anlagerungsprodukten von 1 bis 30 Mol Ethylenoxid an Glycerin;
(b3) Sorbitanmono- und -diester von gesättigten und ungesättigten Fettsäuren mit 6 bis 22 Kohlenstoffatomen und deren Ethylenoxidanlagerungsprodukte;
(b4) Anlagerungsprodukte von 15 bis 60 Mol Ethylenoxid an Ricinusöl und/oder gehärtetes Ricinusöl;
(b5) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(b6) Anlagerungsprodukte von 2 bis 15 Mol Ethylenoxid an Ricinusöl und/oder gehärtetes Ricinusöl;
(b7) Partialester auf Basis linearer, verzweigter, ungesättigter bzw. gesättigter C_{12/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit) sowie Polyglucoside (z.B. Cellulose);
(b8) Trialkylphosphate;
(b9) Wollwachsalkohole;
(b10) Polysiloxan-Polyalkyl-Polyether-Copolymere bzw. entsprechende Derivate;
(b11) Mischester aus Pentaerythrit, Fettsäuren, Citronensäure und Fettalkohol gemäß **DE-PS 11 65 574** und/oder Mischester von Fettsäuren mit 6 bis 22 Kohlenstoffatomen, Methylglucose und Polyolen, vorzugsweise Glycerin sowie
(b12) Polyalkylenglycole.

Die Anlagerungsprodukte von Ethylenoxid und/oder von Propylenoxid an Fettalkohole, Fettsäuren, Alkylphenole, Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und - diester von Anlagerungsprodukten von Ethylenoxid an Glycerin sind aus **DE-PS 20 24 051** als Rückfettungsmittel für kosmetische Zubereitungen bekannt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester und Fettsäurealkanolmide verwendet werden, wobei die letzteren gleichzeitig als Schaumstabilisatoren dienen. Als **Konsistenzgeber** kommen in erster Linie Fettalkohole mit 12 bis 22 und vorzugsweise 16 bis 18 Kohlenstoffatomen in Betracht. Bevorzugt ist eine Kombination dieser Stoffe mit Alkyloligoglucosiden undloder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere XanthanGum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, kationische Stärke, Copolymere von Diallylammoniumsalzen und Acrylamiden, quaternierte Vinylpyrrolidon/Vinylimidazol-Polymere wie z.B. Luviquat® (BASF AG, Ludwigshafen/ FRG), Kondensationsprodukte von Polyglycolen und Aminen, quaternierte Kollagenpolypeptide wie beispielsweise Lauryldimonium hydroxypropyl hydrolyzed collagen (Lamequat®L, Grünau GmbH), quaternierte Weizenpolypeptide, Polyethylenimin, kationische Siliconpolymere wie z.B. Amidomethicone oder Dow Corning, Dow Corning Co./US, Copolymere der Adipinsäure und Dimethylaminohydroxypropyldiethylentrimamin (Cartaretine®, Sandoz/CH), Polyaminopolyamide wie z.B. beschrieben in der **FR-A 22 52 840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese/US, quaternierte Ammoniumsalz-Polymere wie z.B. Mirapol® A-15, Mirapol® AD-1, Mirapol® AZ-1 der Miranol/US.

Geeignete **Siliconverbindungen** sind beispielsweise Dimethylpolysiloxane, Methyl-phenylpolysiloxane, cyclische Silicone sowie amino-, fettsäure-, alkohol-, polyether-, epoxy-, fluor- und/oder alkylmodifizierte Siliconverbindungen, die bei Raumtemperatur sowohl flüssig als auch harzförmig vorliegen können.

Typische Beispiele für **Fette** sind Glyceride, als **Wachse** kommen u.a. Bienenwachs, Paraffinwachs oder Mikrowachse gegebenenfalls in Kombination mit hydrophilen Wachsen, z.B. Cetylstearylalkohol oder Partialglyceriden in Frage. Als **Perlglanzwachse** können insbesondere Mono- und Difettsäureester von Polyalkylenglycolen, Partialglyceride oder Ester von Fettalkoholen mit mehrwertigen Carbonsäuren bzw. Hydroxycarbonsäuren verwendet werden. Als **Stabilisatoren** können Metallsalze von Fettsäuren wie z.B. Magnesium-, Aluminium- und/oder Zinkstearat eingesetzt werden. Unter **biogenen Wirkstoffen** sind beispielsweise Bisabolol, Allantoin, Phytantriol, Panthenol, AHA-Säuren, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Zur Verbesserung des Fließverhaltens können ferner **Hydrotrope** wie beispielsweise Ethanol, Isopropylalkohol, Propylenglycol oder Glucose eingesetzt werden. Als **Konservierungsmittel** eignen sich beispielsweise Phenoxyethanol, Formaldehydlösung, Parabene, Pentandiol oder Sorbinsäure. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

### I. Anwendungstechnische Beispiele

Zur Beurteilung der dermatologischen Veträglichkeit wurde der transepidermale Wasser-verlust an einer Schweineepidermis untersucht. Hierzu wurden definierte Hautstücke bei 40°C über einen Zeitraum von 30 min mit den verschiedenen Testlösungen behandelt und der TEWL-Wert gravimetrisch bestimmt. Bei den Testlösungen handelte es sich um Mischungen von 17 Gew.-% Plantaren® PS 10 (Dodecyl Polyglucose and Sodium Laureth Sulfate) und 1,5 Gew.-% Fettsäurepartialglycerid (Wasser ad 100 Gew.-%). Die Ergebnisse sind in Tabelle 2 zusammengefaßt. Als TEWL-Wert wird hierbei das in Prozent ausgedrückte Verhältnis des transepidermalen Wasserverlustes einer unbehandelten zu einer behandelten Probe angegeben. Je niedriger der Wert ist, um so besser ist die dermatologische Verträglichkeit.

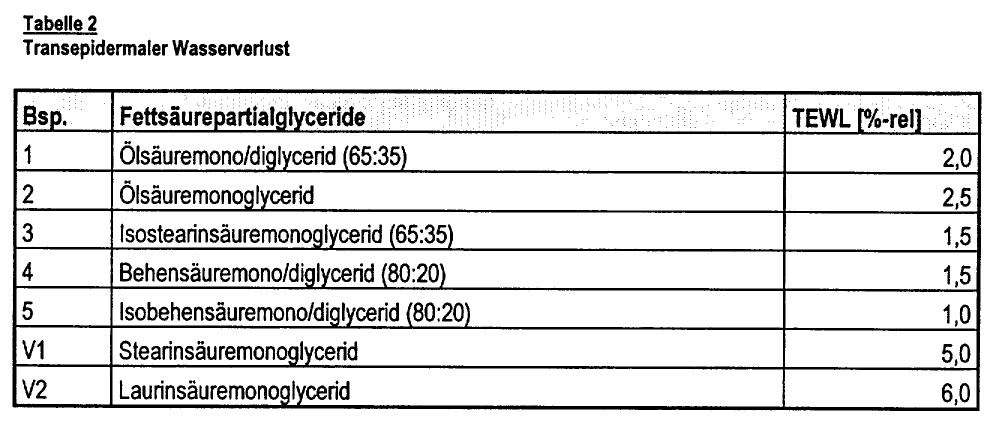

Die Beispiele und Vergleichsbeispiele zeigen deutlich, daß die erfindungsgemäßen Zubereitungen einen wesentlich geringeren transepidermalen Wasserverlust bewirken als die Vergleichsmischungen und somit eine signifikant bessere dermatologische Verträglichkeit besitzen.

### II. Rezepturbeispiele

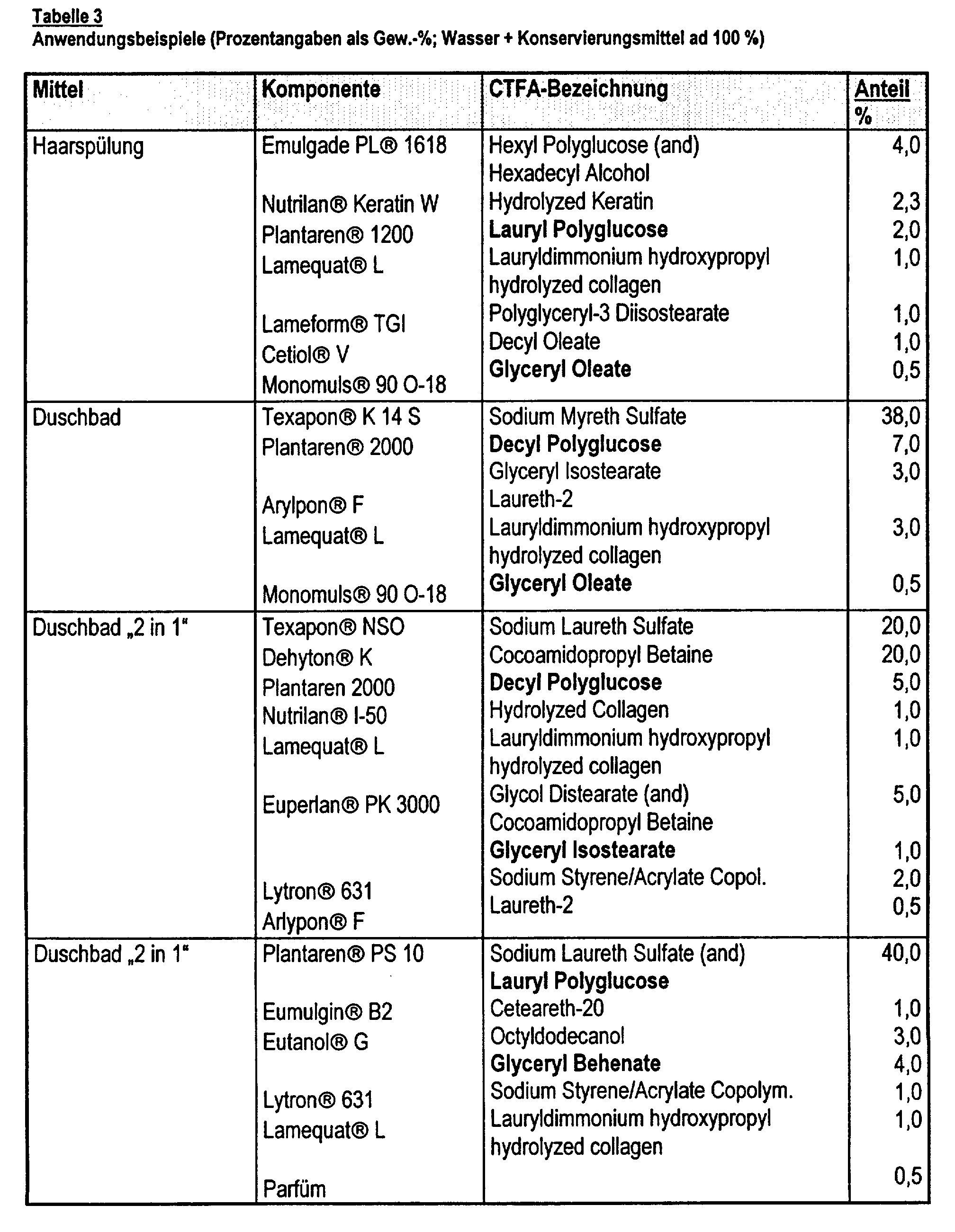

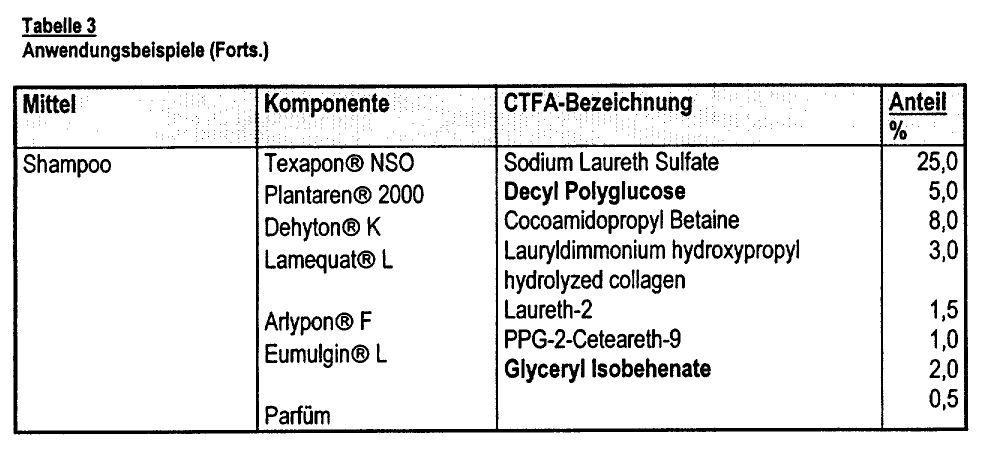

## Patentansprüche

1. Kosmetische und/oder pharmazeutische Zubereitungen, enthaltend
(a) 0,5 bis 70 Gew.-% Zuckertenside ausgewählt aus der Gruppe, die gebildet wird von (a1) Alkyl- und/oder Alkenyloligoglykosiden und/oder (a2) Fettsäure-N-alkylpolyhydroxyalkylamiden, und
(b) 0,5 bis 40 Gew.-% Fettsäurepartialglyceride ausgewählt aus der Gruppe, die gebildet wird von Ölsäuremonoglycerid, Ölsäurediglycerid, Isostearinsäuremonoglycerid, Isostearinsäurediglycerid, Behensäuremonoglycerid, Behensäurediglycerid, Isobehensäuremonoglycerid und/oder Isobehensäurediglycerid,
mit der Maßgabe, daß sich die Mengenangaben gegebenenfalls mit üblichen Hilfs- und Zusatzstoffen und/oder Wasser zu 100 Gew.-% ergänzen.

2. Zubereitungen nach Anspruch 1, **dadurch gekennzeichnet,** daß sie Alkyl- und Alkenyloligoglykoside der Formel **(I)** enthalten,
**R**^{**1**}**O-[G]**_{**p**} **(I)**
in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

3. Zubereitungen nach den Ansprüchen 1 und 2, **dadurch gekennzeichnet,** daß sie Fettsäure-N-alkylpolyhydroxyalkylamide der Formel **(II)** enthalten, 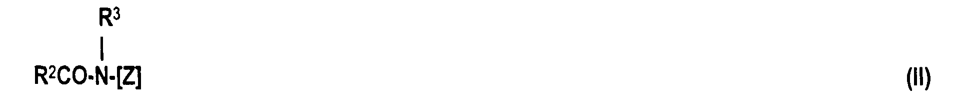 in der R²CO für einen aliphatischen Acylrest mit 6 bis 22 Kohlenstoffatomen, R³ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

4. Zubereitungen nach den Ansprüchen 1 bis 3, **dadurch gekennzeichnet,** daß sie weiterhin 0,5 bis 40 Gew.-% anionische und/oder amphotere bzw. zwitterionische Tenside enthalten.

5. Zubereitungen nach den Ansprüchen 1 bis 4, **dadurch gekennzeichnet**, daß sie weiterhin 0,5 bis 25 Gew.-% Ölkörper enthalten.

6. Zubereitungen nach den Ansprüchen 1 bis 5, **dadurch gekennzeichnet**, daß sie weiterhin 0,5 bis 25 Gew.-% Glycerin und/oder Wasser enthalten.

7. Verwendungen von Zubereitungen nach Anspruch 1 als Rückfettungsmittel zur Herstellung von kosmetischen und pharmazeutischen Produkten.

## Claims

1. Cosmetic and/or pharmaceutical formulations containing
(a) 0.5 to 70% by weight of sugar surfactants selected from the group consisting of (a1) alkyl and/or alkenyl oligoglycosides and/or (a2) fatty acid-N-alkyl polyhydroxyalkylamides and
(b) 0.5 to 40% by weight of fatty acid partial glycerides selected from the group consisting of oleic acid monoglyceride, oleic acid diglyceride, isostearic acid monoglyceride, isostearic acid diglyceride, behenic acid monoglyceride, behenic acid diglyceride, isobehenic acid monoglyceride and/or isobehenic acid diglyceride,
with the proviso that the quantities shown add up to 100% by weight, optionally with typical auxiliaries and additives and/or water.

2. Formulations as claimed in claim 1, characterized in that they contain alkyl and alkenyl oligoglycosides corresponding to formula (I):
**R**^{**1**}**O-[G]**_{**p**} **(I)**
in which R¹ is an alkyl and/or alkenyl radical containing 4 to 22 carbon atoms, G is a sugar unit containing 5 or 6 carbon atoms and p is a number of 1 to 10.

3. Formulations as claimed in claims 1 and 2, characterized in that they contain fatty acid-N-alkyl polyhydroxyalkylamides corresponding to formula (II): 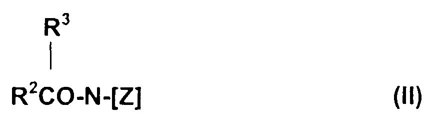 in which R²CO is an aliphatic acyl radical containing 6 to 22 carbon atoms, R³ is hydrogen, an alkyl or hydroxyalkyl radical containing 1 to 4 carbon atoms and [Z] is a linear or branched polyhydroxyalkyl radical containing 3 to 12 carbon atoms and 3 to 10 hydroxyl groups.

4. Formulations as claimed in claims 1 to 3, characterized in that they additionally contain 0.5 to 40% by weight of anionic and/or amphoteric or zwitterionic surfactants.

5. Formulations as claimed in claims 1 to 4, characterized in that they additionally contain 0.5 to 25% by weight of oils.

6. Formulations as claimed in claims 1 to 5, characterized in that they additionally contain 0.5 to 25% by weight of glycerol and/or water.

7. The use of the formulations claimed in claim 1 as refatting agents for the production of cosmetic and pharmaceutical products.

## Revendications

1. Préparations cosmétiques et/ou pharmaceutiques contenant :
a) de 0,5 à 70 % en poids d'agents tensioactifs à base de sucre sélectionnés dans le groupe qui est formé de :
a1) alkyl- et/ou alkényloligoglucosides et/ou
a2) des N-alkylpolyhydroxyalkylamides d'acide gras et,
b) de 0,5 à 40 % en poids de glycérides partiels d'acides gras sélectionnés dans le groupe qui est formé du monoglycéride d'acide oléique, du diglycéride d'acide oléique, du monoglycéride d'acide isostéarique, du diglycéride d'acide isostéarique, du monoglycéride d'acide behenique, du monoglycéride d'acide isobéhénique et/ou du diglycéride d'acide isobéhénique.
avec la restriction que les indications de quantités se complètent à 100 % en poids, avec les adjuvants et les additifs usuels et/ou l'eau.

2. Préparations selon la revendication 1,
caractérisées en ce qu'
elles renferment des alkyl et des alkényloligoglycosides de formule (I),
R¹O-[G]ₚ (I)
dans laquelle R¹ représente un radical alkyle et/ou alkényle ayant de 4 à 22 atomes de carbone, G représente un radical de sucre ayant 5 ou 6 atomes de carbone et p représente un nombre allant de 1 à 10.

3. Préparations selon les revendications 1 et 2,
caractérisées en ce qu'
elles renferment des N-alkylpolyhydroxyalkylamides de formule (II), 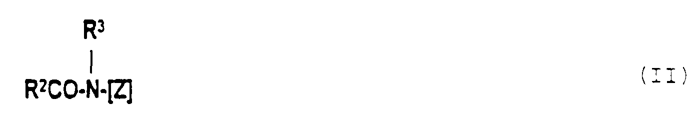 dans laquelle R²CO représente un radical acyle aliphatique, ayant de 6 à 22 atomes de carbone, R³ représente un radical alkyle ou hydroxyalkyle ayant de 1 à 4 atomes de carbone, et [Z] représente un radical polyhydroxyalkyle linéaire ou ramifié, ayant de 3 à 12 atomes de carbone, et de 3 à 10 groupes hydroxyle.

4. Préparations selon les revendications 1 à 3,
caractérisées en ce qu'
elles renferment en outre de 0,5 à 40 % en poids d'agents tensioactifs anioniques et/ou amphotères ou zwitterioniques.

5. Préparations selon les revendications 1 à 4,
caractérisées en ce qu'
elles renferment en outre de 0,5 à 25 % en poids de composés huileux.

6. Préparations selon les revendications 1 à 5,
caractérisées en ce qu'
elles renferment en outre de 0,5 à 25 % en poids de glycérol et/ou d'eau.

7. Utilisations des préparations selon la revendication 1 comme agent de graissage en retour en vue de la préparation de produits cosmétiques et pharmaceutiques.
